# EUROPEAN PATENT APPLICATION

(11) **EP 3 909 592 A1**
(43) Date of publication of application: **17.11.2021**
(21) Application number: 20174461.2
(22) Date of filing: 13.05.2020
(51) Int. Cl.: A61K 35/28, C12N 5/0775, A61P 31/14

(54) **TREATMENT OF COMPLICATIONS CAUSED BY INFECTIONS OF SEVERE ACUTE RESPIRATORY SYNDROME CORONAVIRUS**

(71) Applicant: Johann Wolfgang Goethe-Universität Frankfurt am Main, 60323 Frankfurt am Main (DE)
(72) Inventor: BADER, Peter, 63303 Dreieich (DE); SONNTAGBAUER, Michael, 60594 Frankfurt am Main (DE); NEB, Holger, 65239 Hochheim (DE); ZACHAROWSKI, Kai, 61350 Bad Homburg (DE); KUCI, Selim, 60599 Frankfurt am Main (DE); BÖNIG, Halvard, 60437 Frankfurt (DE); HÜNECKE, Sabine, 60385 Frankfurt am Main (DE); KUCI, Zyrafete, 60599 Frankfurt am Main (DE)
(74) Representative: Kuttenkeuler, David

(57) **Abstract**

The invention is based on the finding that a subjects suffering from a viral infection and which is distinguished by a systemic inflammatory complication such as systemic endotheliitis are in particular indicated for a treatment with mesenchymal stromal cell (MSC) compositions. The invention provides treatments in the context of SARS Cov2 infections, in severely affected subjects receiving mechanical ventilation. Provided are MSC compositions for use in treatments, diagnostic stratification methods, and diagnostic kits for such purposes.

## Description

### FIELD OF THE INVENTION

The invention is based on the finding that a subjects suffering from a viral infection and which is distinguished by a systemic inflammatory complication such as systemic endotheliitis are in particular indicated for a treatment with mesenchymal stromal cell (MSC) compositions. The invention provides treatments in the context of SARS Cov2 infections, in severely affected subjects receiving mechanical ventilation. Provided are MSC compositions for use in treatments, diagnostic stratification methods, and diagnostic kits for such purposes.

### DESCRIPTION

Whereas the pandemic due do Covid-19 continues to spread, the severe acute respiratory syndrome coronavirus 2 (SARS-CoV-2) causes inflammation in more than 30% of patients with a 30%-60% mortality rate for those requiring hospitalization in an intensive care unit. The pathophysiology of the entire Covid-19 disease is not yet understood. Dramatic inflammation syndrome often results in severe endotheliitis which in turn might be the initial step in inducing multi organ affection and multi organ failure. One of the most frequent manifestations is an acute pulmonary inflammation which leads consecutively to the clinical picture for acute respiratory distress syndrome (ARDS). This causes acute respiratory failure, necessitating intubation, mechanical ventilation and often the need for extracorporal oxygenation (ECMO). The prognosis for the patients is poor. Mortality rates of up to 90% are reported. Currently, there is no treatment.

Mesenchymal stromal cells (MSC) feature several attractive characteristics and especially their known anti-inflammatory, anti-fibrotic and immunomodulatory properties render them to be tested as immunomodulatory therapy in these patients. In Germany the mesenchymal stromal cell product Obnitix® is liscensed for the treatment of acute steroid refractory graft versus host disease (GVHD) according to §4b AMG. These cells have already been given to more than 250 patients in Germany and Europe with an excellent safety record.

International publication No. WO 2016/008895 discloses a MSC preparations and a method of obtaining MSC preparations that are based on a pooling of bone marrow samples from genetically distinct donors and a subsequent isolation of the MSCs. The MSC isolated according to this methods show favourable immune modulatory characteristics and preferably used in the context of GVHD.

Thus, it is an objection of the invention to provide treatment options for subject suffering from severe complications during viral infections.

### BRIEF DESCRIPTION OF THE INVENTION

Generally, and by way of brief description, the main aspects of the present invention can be described as follows:

In **a first aspect,** the invention pertains to a cellular composition for use in the treatment of a disorder associated with a viral infection in a subject, the cellular composition comprising mesenchymal stromal cells (MSC), and wherein the treatment comprises the administration of a therapeutically effective amount of the cellular composition to the subject.

In **a second aspect,** the invention pertains to a method for stratifying a subject suffering from a disorder associated with a viral infection for a treatment with mesenchymal stromal cells (MSC), the method comprising (i) providing a biological sample of the subject; (ii) determining in the biological sample one or more marker(s) of systemic inflammation; wherein the subject is stratified for a treatment with MSC if the determined one or more marker(s) of system inflammation indicates a systemic inflammation in the subject.

In **a third aspect,** the invention pertains to a kit for stratifying a subject suffering from a disorder associated with a viral infection for a treatment with mesenchymal stromal cells (MSC), comprising means for determining one or more marker(s) of systemic inflammation.

In **a fourth aspect,** the invention pertains to a use of a kit in a method for stratifying a subject suffering from a disorder associated with a viral infection for a treatment with mesenchymal stromal cells (MSC), wherein the kit comprises means for determining one or more marker(s) of systemic inflammation.

In **a fifth aspect,** the invention pertains to a method for the treatment of a disorder associated with a viral infection in a subject, comprising a step of administering to the subject a therapeutically effective amount of a mesenchymal stromal cell (MSC) preparation.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **a first aspect,** the invention pertains to a cellular composition for use in the treatment of a disorder associated with a viral infection in a subject, the cellular composition comprising mesenchymal stromal cells (MSC), and wherein the treatment comprises the administration of a therapeutically effective amount of the cellular composition to the subject.

The present invention is based on the surprising finding that a specific group of severely affected patients suffering from complications in course of a viral disease, for example COVID-19, caused by SARS-Cov2.

The term "cellular composition" refers to a preparation of cells, which preparation may include, in addition to the cells, non-cellular components such as cell culture media, e.g. proteins, amino acids, nucleic acids, nucleotides, co-enzyme, anti-oxidants, metals and the like. Furthermore, the cellular composition can have components which do not affect the growth or viability of the cellular component, but which are used to provide the cells in a particular format, e.g., as polymeric matrix for encapsulation or as a pharmaceutical preparation.

In the present disclosure, the term "mesenchymal stromal cell" or "MSC" is a multipotent stromal cell that can differentiate into a variety of cell types, including, but not limited to osteoblasts (bone cells), chondrocytes (cartilage cells), myocytes (muscle cells) and adipocytes (fat cells). These cells are also known as "mesenchymal stem cells" due to their multipotency. This biologically important cell population is able to support hematopoiesis, can differentiate along mesenchymal and non-mesenchymal lineages *in vitro,* is capable of suppressing alloresponses and appear to be non-immunogenic. These cells are known to either secrete certain proteins and/or compounds, which is turn influence the microenvironment around the cells, or when not directly expressing the proteins themselves, mesenchymal stromal cells are known to influence the downstream expression of other proteins, which for example, may be affected by the presence or absence of a mesenchymal stromal cell. That is to say that the presence of a mesenchymal stromal cell within the environment of another cell can cause the other cell to start producing, or even secreting, certain proteins. Therefore, disclosed herein are both compounds and/or proteins secreted by the mesenchymal stromal cells themselves and are therefore considered to be derived from MSCs as they are specifically obtained from MSCs, as well as proteins and compounds that are produced and/or secreted as a result of the presence of mesenchymal stromal cells within the cell environment. Included therein is also the downstream expression of proteins that is initiated via one or more expression cascades due to the presence of mesenchymal stromal cells in the microenvironment.

In preferred embodiments of the invention, the MSCs (such as Obnitix®) do not express, or at least have a reduced expression of, ACE-2 receptors. This is because ACE-2 receptors are considered as entry molecules for SARS-CoV-2 in the cells. In addition, in some embodiments, the MSC usable in the present context express only minimal levels of tissue factor (CD142 antigen), which is in contrast to adipose-derived or umbilical cord- derived mesenchymal stromal cells which express high levels of CD142 which elicits an instant blood-mediated inflammatory reaction (IBMIR) resulting in thrombotic complications. Hence, CD142 expressing MSCs are in preferred embodiments excluded.

In preferred embodiments of the invention the MSC are a composition of cells obtained from genetically distinct donors, such as donors that are not identical twins or are not, in the case of animal donors, individuals from an isogenic population. The term "genetically distinct" as used herein, indicates that at least one difference at the genomic level exists between bone marrow donors/subjects. Bone marrow can be collected from 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100 or more donors. Most preferred is the use of 4 to 8 different donor samples.

Most preferably the polygenic MSC of the invention are obtained according to the methods described in WO 2016/008895 which is incorporated herein by reference in its entirety, and in particular incorporated is the disclosure of the MSC isolation method on pages 5, 2^{nd} paragraph to page 8, 3^{rd} paragraph.

The terms "treatment," "treating," "treat," and the like are used herein to generally refer to obtaining a desired pharmacologic and/or physiologic effect. The effect may be prophylactic (also referred to as "prevention" or "preventing") in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject who may be predisposed to the disease or symptom but has not yet been diagnosed as having it; (b) inhibiting the disease symptom, i.e., arresting its development; (c) relieving the disease symptom, i.e., causing regression of the disease or symptom; (d) limiting spread of a virus from one cell to another within an individual, e.g., limiting spread of a virus from an infected epithelial cell to other, uninfected, epithelial cells within an individual; (e) limiting replication of a virus within an individual; (f) limiting entry of a virus into a cell in an individual; and (g) reducing the number of viruses in an individual or in a target tissue or target biological sample in an individual.

The terms "subject," "individual," "host," and "patient" are used interchangeably herein to refer to a mammal, including, but not limited to, murines (rats, mice), felines, non-human primates (e.g., simians), humans, canines, ungulates, etc. In some embodiments, a "subject" is a human, and can also be referred to as a "patient."

A "therapeutically effective amount" or "efficacious amount" means the amount of a compound (such as a cellular (MSC) composition) that, when administered to a mammal or other subject for treating a disease, is sufficient to effect such treatment for the disease. The "therapeutically effective amount" will vary depending on the compound, the disease and its severity and the age, weight, etc., of the subject to be treated. In context of the herein disclosed invention a therapeutically effective amount of MSC comprises 10⁵ to 10⁷, preferably about (+/-20%) 1x10⁶ MSC/kg ideal body weight. In some preferred embodiments, the therapeutically effective amount does not exceed 90x10⁶ MSC/kg body weight for each subject during the treatment.

The present disclosure provides methods of treating a viral infection in a subject. The uses and methods generally involve administering to a subject in need thereof an effective amount of a cellular (MSC) composition. The present disclosure further provides methods of treating virus-induced acute exacerbation of a chronic lung disease, the methods generally involving administering to an individual in need thereof (e.g., an individual having a chronic lung disease) an effective amount of a cellular (MSC) composition.

The present disclosure provides compounds for use in, and methods for, treating complications caused by a virus infection, where the virus is a member of the Coronaviridae family, the method involving administering an effective amount of a cellular (MSC) composition to an individual infected with a member of the Coronaviridae family. Coronaviridae includes, e.g., coronaviruses, e.g., human coronavirus 229E (HCoV- 229E), human coronavirus OC43 (HCoV-OC43), and SARS-CoV (the causative agent of severe acute respiratory syndrome (SARS)), which cause upper respiratory tract infection, lower respiratory tract infections, and gastroenteritis.

Irrespective of the above, in all of the disclosed aspects and embodiments, the invention is particular useful for the treatment or prevention of complications, preferably systemic inflammation or endotheliitis, caused by viral infection caused by SARS Cov-2 - thus the invention seeks in particular embodiments to provide a treatment for COVID-19 related complications, and any related disorders, the method involving administering an effective amount of a cellular (MSC) composition to an individual infected with SARS-CoV-2.

In any one of the above embodiments, the individual is a human of from about one month to about 6 months, from about 6 months to about 1 year, from about 1 year to about 5 years, from about 5 years to about 12 years, from about 13 years to about 18 years, from about 18 years to about 25 years, from about 25 years to about 50 years, from about 50 years to about 75 years of age, or older than 75 years of age. In some embodiments, the individual has a chronic lung disease (e.g., emphysema, chronic bronchitis, asthma, cystic fibrosis, bronchiectasis, COPD, or interstitial lung disease). In some embodiments, the individual has, in addition to a coronavirus infection, pneumonia, where the pneumonia is caused by the coronavirus (preferably SARS-CoV-2) or by another viral or bacterial infection. In some embodiments the human subject is immune-compromised.

The disorder treated in accordance with the invention is preferably an inflammatory complication associated with a respiratory disorder caused by the viral infection in the subject. Such respiratory disorder is in some instances pneumonia or severe pneumonia, such as acute respiratory distress syndrome (ARDS), preferably an ARDS associated with lung injury.

An "inflammatory complication" in context of a viral infection in accordance with the disclosure is for example a systemic inflammation and preferably is systemic endotheliitis. The term "endotheliitis" in context of the herein disclosed invention shall refer to an inflammation of the endothelium preferably the vascular endothelium - in context of the invention preferably the vascular endothelium of the lung blood vessel system. If such vascular endothelial inflammation is noticed in systemic scope, the invention will refer to the condition as a "systemic endotheliitis".

In context of the invention, preferably, the presence of inflammation and endotheliitis in the subject is characterized by one or more of the following serum markers before administration of the MSC:
- Leucocytosis; preferably of 3 - 15, more preferably a leucocyte [plasma] count of 4 - 12, and most preferably 4.2 - 10.8 cells per nl;
- Elevated c reactive protein levels, preferably of > about 5 mg/dl preferably of > about 10,5 mg/dl;
- Normal or Elevated Interleukin (IL) - 6 levels;
- Elevated HLA-DR activated cytotoxic CD8 positive T-cells;
- Normal or Elevated procalcitonin (PCT) levels; and/or;
- Normal or elevated lactat dehydrogenase (LDH) levels;
- An increased capillary refill time, such as equal to, or more than, about 2 seconds, equal to, or more than, about 2.5 seconds, and preferably equal to, or more than, about than about 3 seconds.

In some embodiments of the invention the treatment comprises a reduction of a cell mediated immune response in the subject, preferably comprises a reduction of leucocytosis in the subject and preferably involves a reduction of activated CD3+/CD8+ T cells in the subject.

In context of the present invention the MSC therapy is in particular useful for a subject which is distinguished by a severe virally caused disease. The subject of the invention is preferably distinguished by any one or a combination of, or all of, the following criteria:
**(A)** The subject is distinguished in that it receives mechanical ventilation.
**(B)** The subject is distinguished by showing signs of micro capillary failure (or microcirculatory dysfunction), preferably defined as an increased capillary refill time, such as equal to, or more than, about 3 seconds, about 2.5 seconds, and preferably more than 2 seconds. Capillary refill time (CRT) is defined as the time taken for colour to return to an external capillary bed after pressure is applied to cause blanching. It can be measured by holding a hand higher than heart-level and pressing the soft pad of a finger or fingernail until it turns white, then taking note of the time needed for the colour to return once pressure is released. Preferably, the capillary refill time is measured using the above described "Capillary nail refill test". Normal capillary refill time is usually less than 2 seconds. Capillary refill time can also be assessed in animals by pressing on their gums as opposed to the sternum which is generally covered with fur or inaccessible.
**(C)** The subject is distinguished by a pₐO₂/FᵢO₂ of less than about 250, 220, 200, 180, 150, 130, 100, or 80 and preferably less than 150 mmHg while showing a positive end-expiratory pressure (PEEP) of equal to, or more than, 4, 5, 6, 7 and preferably 8 cmH₂O. The pₐO₂/FᵢO₂ ratio is the ratio of arterial oxygen partial pressure (pₐO₂ in mmHg) to fractional inspired oxygen (Fi02 expressed as a fraction, not a percentage). The acronym PEEP as used in this case represents the term Positive End Expiratory Pressure, which is the aforementioned resistance to exhaled airflow at a preset pressure. The application of PEEP has long been recognized as a benefit to patients of cardiopulmonary resuscitation by maintaining a degree of inflation in the lungs, thus enabling a prolonged contact between the inhaled gases and the subject's pulmonary capillary bed, and preventing collapse of the lung.
**(D)** The subject is distinguished by an ongoing veno-venous / veno-arterial extra-corporal life support, preferably the subject is distinguished in that it receives extracorporeal membrane oxygenation (ECMO). Severely affected patients that are under ECMO support according to the invention are preferred subjects for treatment. ECMO provides temporary life support to patients usually with reversible cardiac or respiratory failure. The method delivers oxygen by extracorporeal measures by mechanical bypass that takes place outside the body of the person being treated. It is generally designed or intended for a patient whose heart and lungs cannot perform normally on their own.

In some embodiments the subject is distinguished by any one of the aforementioned criteria A to D, in some embodiments, the subject is distinguished by at least two, or preferably three, or preferably all of the criteria.

In some preferred embodiments, the subject is concomitantly or sequentially treated with at least one further antiviral therapy. For example, the treatment further comprises administering to the subject an anti-viral agent, preferably remdesivir.

In **a second aspect,** the invention pertains to a method for stratifying a subject suffering from a disorder associated with a viral infection for a treatment with mesenchymal stromal cells (MSC), the method comprising (i) providing a biological sample of the subject; (ii) determining in the biological sample one or more marker(s) of systemic inflammation; wherein the subject is stratified for a treatment with MSC if the determined one or more marker(s) of system inflammation indicates a systemic inflammation in the subject.

In some preferred embodiments, the subject to be stratified for a treatment with MSC is further characterized by showing an increased capillary refill time, such as equal to, or more than, about 2 seconds, about 2.5 seconds, and preferably equal to, or more than about 3 seconds.

A systemic inflammation is a systemic endotheliitis as described herein elsewhere.

In **a third aspect,** the invention pertains to a kit for stratifying a subject suffering from a disorder associated with a viral infection for a treatment with mesenchymal stromal cells (MSC), comprising means for determining one or more marker(s) of systemic inflammation.

In **a fourth aspect,** the invention pertains to a use of a kit in a method for stratifying a subject suffering from a disorder associated with a viral infection for a treatment with mesenchymal stromal cells (MSC), wherein the kit comprises means for determining one or more marker(s) of systemic inflammation.

In **a fifth aspect,** the invention pertains to a method for the treatment of a disorder associated with a viral infection in a subject, comprising a step of administering to the subject a therapeutically effective amount of a mesenchymal stromal cell (MSC) preparation.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:
**Figure 1****:** shows **a CT 15.04.2020** Pronounced assymetrical bipulmonary consolidations. Left lung 75-90% affected, right lung 50-75% affected. Bilateral anterior lung segments with ground glass opacities. No pleural effusion.
**Figure 2****:** shows an X-Rays of the patient on different dates - 20.04.2020: Generalized peripheral opacities, most pronounced of the lower and middle segment of the left lung consistent with COVID-19; 22.04.2020: Diminishing opacities of the right lung. Persistent hazy opacities of the lower and middle segment of the right; 24.04.2020: Consistent peripheral opacities; 30.04.2020: Diminishing opacities of the right lung. Persistent opacities of the lower and middle segment of the right. Without ECMO catheter.
**Figure 3**: shows course of leukocytes after administration of MSC
**Figure 4**: shows course of procalcitonin after administration of MSC
**Figure 5**: shows course of transaminases after administration of MSC
**Figure 6****:** shows course of lactat dehydrogenase after administration of MSC
**Figure 7**: shows the monitoring of immune-status in a patient suffering from Covid19 before, one, four and eight days after MSC immunotherapy.
**Figure 8****:** shows HLA DR staining of monocytes

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Mesenchymal Stromal Cell Therapy of Severe COVID-19/Endotheliitis Patient

The first patient who received MSC was a 29 year old female patient with a body weight of 120 kg. She developed first signs of Covid-19 disease on April 1st with fever and reduced general condition. On April 5th, she needed intubation and mechanical ventilation. Due to her non-controllable inflammation she continuously deteriorated and was put on extracorporeal mechanical oxygenation (ECMO) on April 13th. In desperate situation and after informed consent by her family, she received one dose of MSC on April 20th.

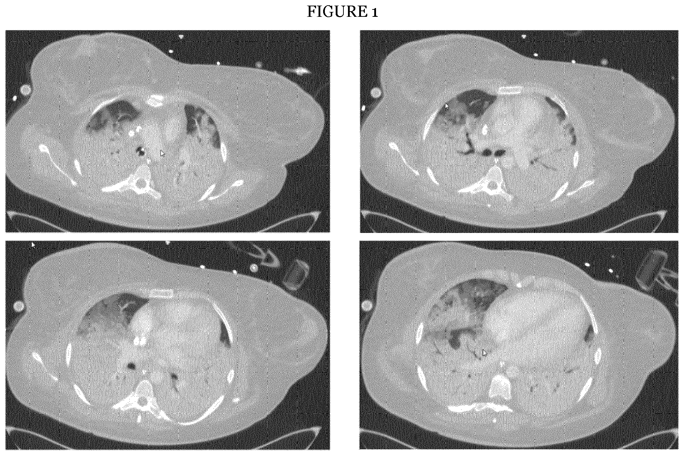

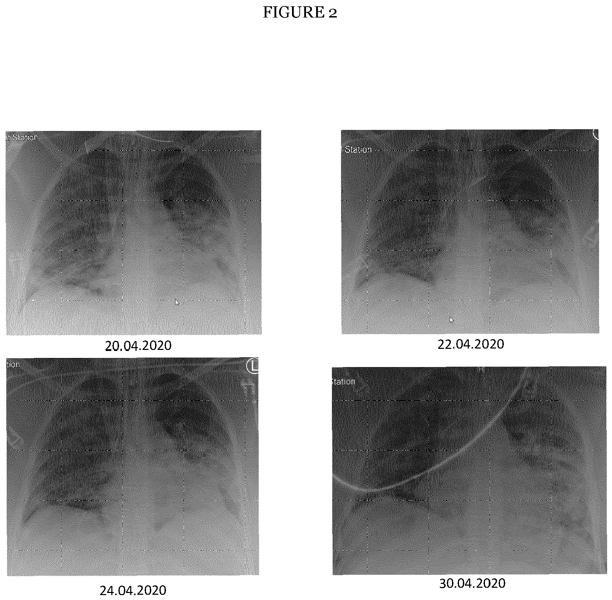

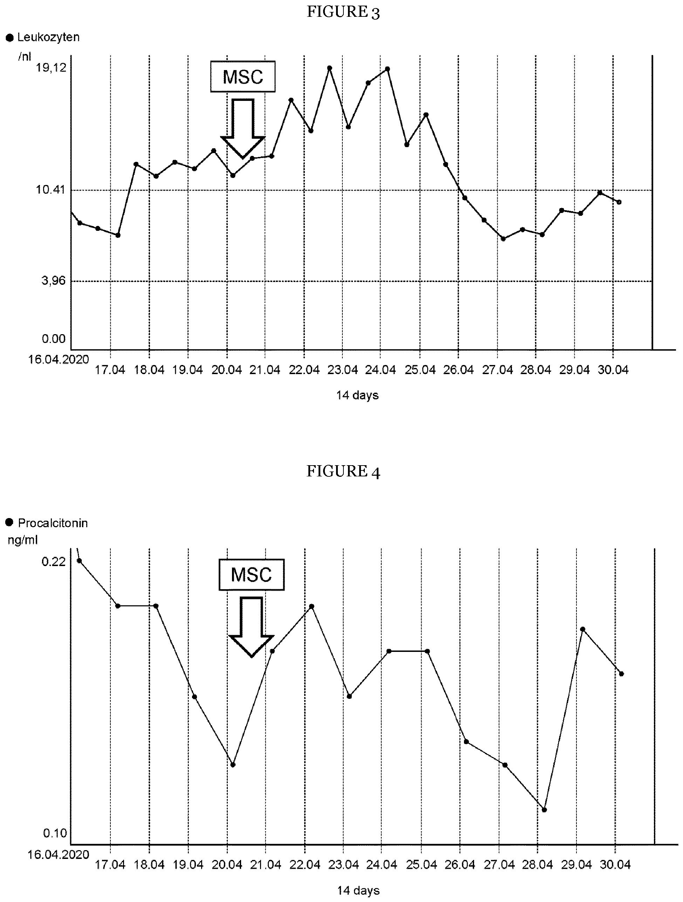

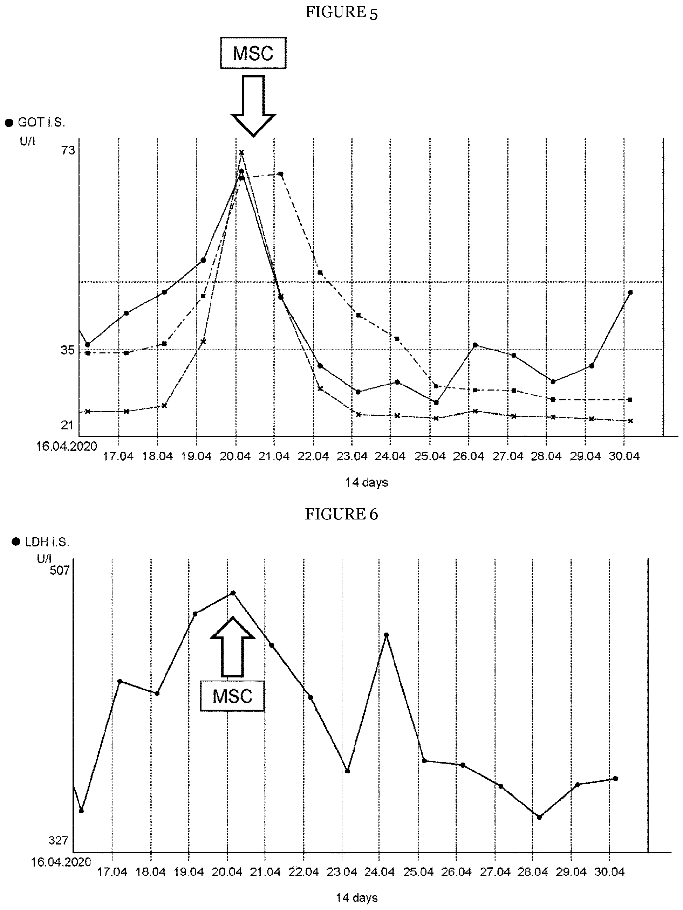

This modulation of the patient's inflammation went along with a reduction of inflammatory laboratory values and rapid and sustained improvement of the patient's clinical condition (Figures 1 and 2 and 3 to 6). Both ECMO support could be steadily reduced the patient lungs took continuously over oxygen saturation. ECMO support could be terminated one week later on April 30th.

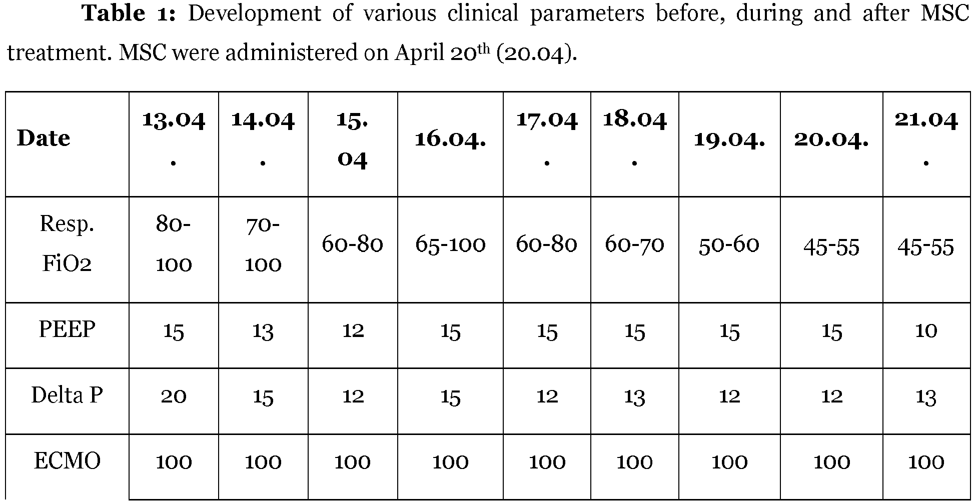

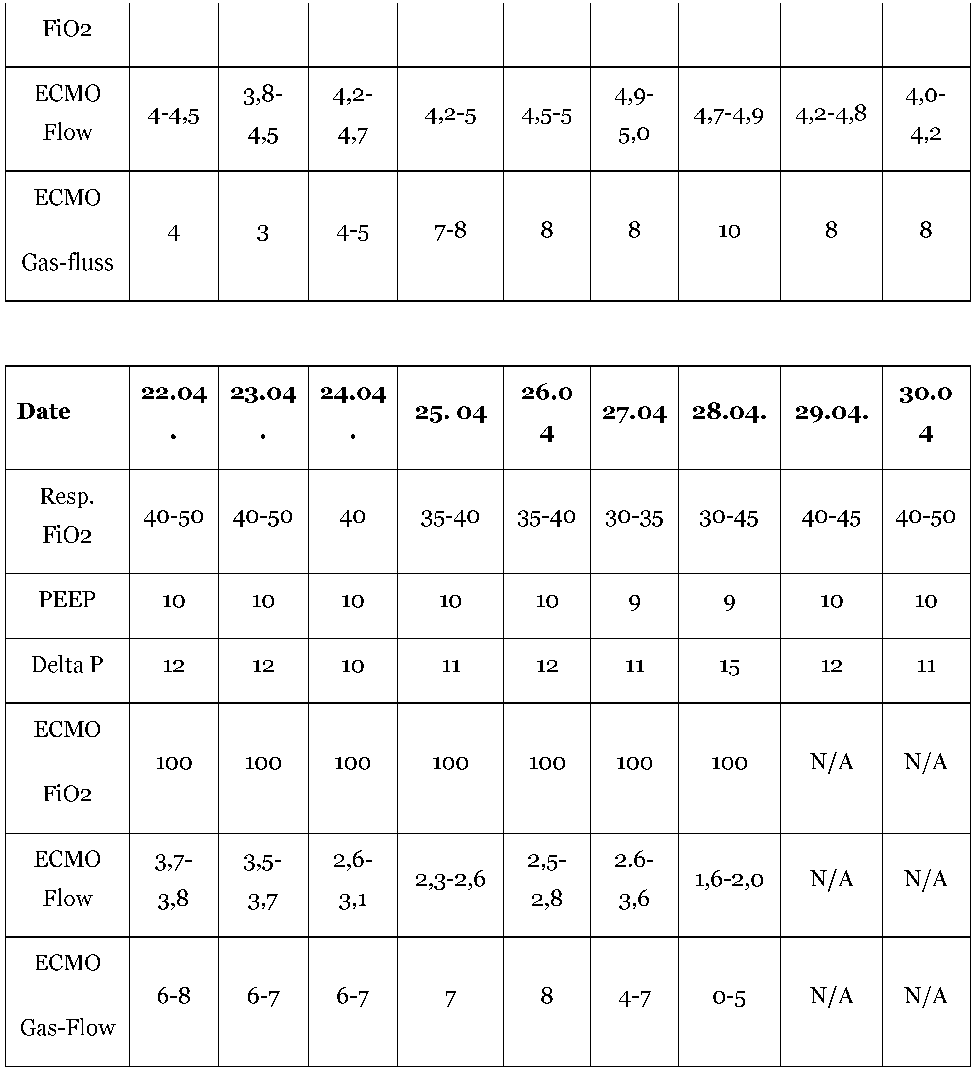

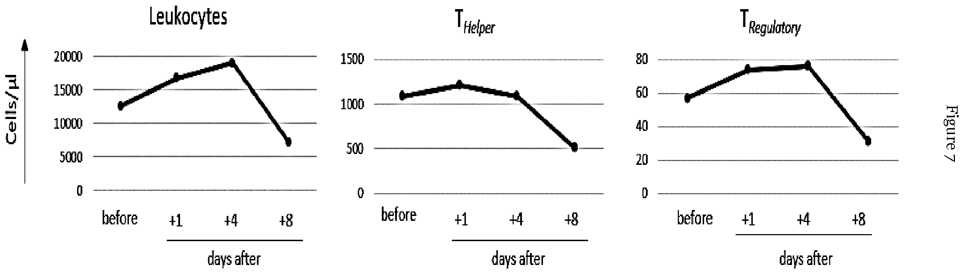

The patient received one dose of MSC on April 20th. By the time the patient had clinical sings of inflammation with leukocytosis, elevated CRP, IL-6 and PCT as well as HLA-DR activated cytotoxic CD8 T-cells. After transfusion of one dose of MSC, the patient's regulatory CD4 T cells temporarily increased, leading to reduction of activated T-cells (Figure 7).

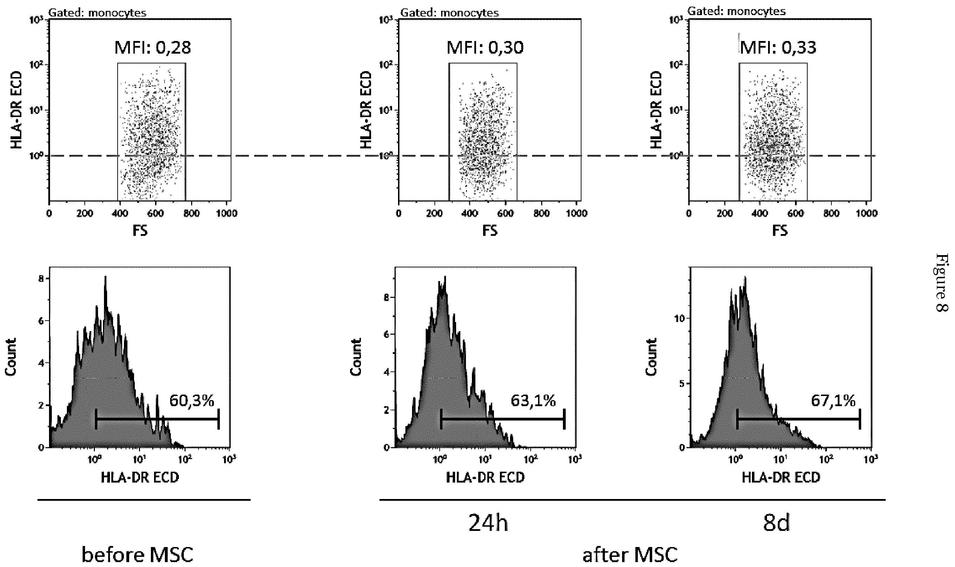

In patients suffering from bacterial sepsis, a long lasting decline of HLA-DR expression was related to poor survival. Therefore, we investigated HLA-DR activation on monocytes with the result of a slight increase in the percentage and mean fluorescence intensity of HLA-DR expression on monocytes 24h and eight days following Obnitix® application indicating effective immune modulation of Obnitix® (Figure 8).

### Example 2 [prophetic]: Clinical Study of Mesenchymal Stromal Cell Therapy of COVID-19 Patients

A study is conducted to investigate whether treatment with MSC-FFm (Obnitix®) in patients with critically severe Covid-19 disease will reduce mechanical ventilation times and thereby improve survival.

The study is conducted according the following summary in table 2:

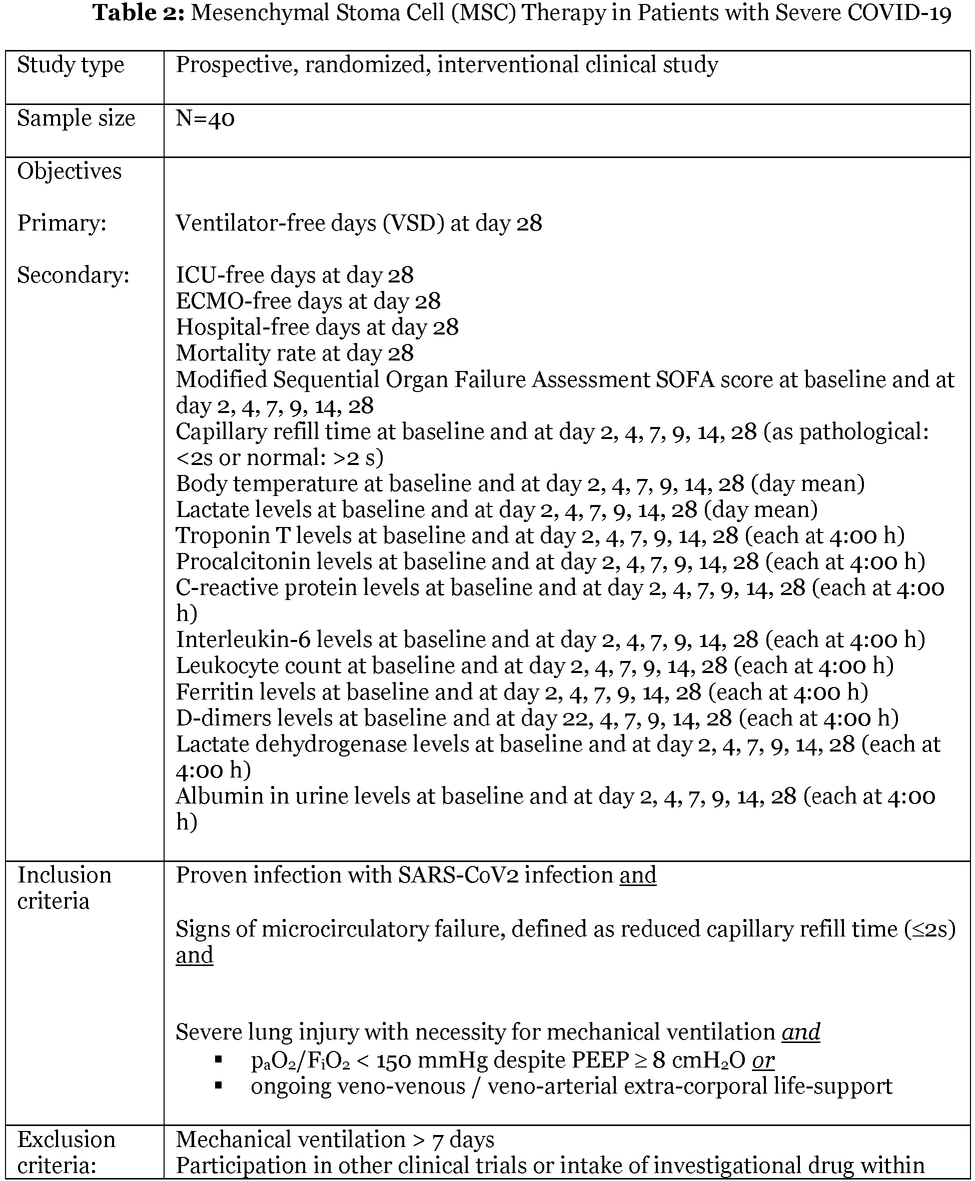

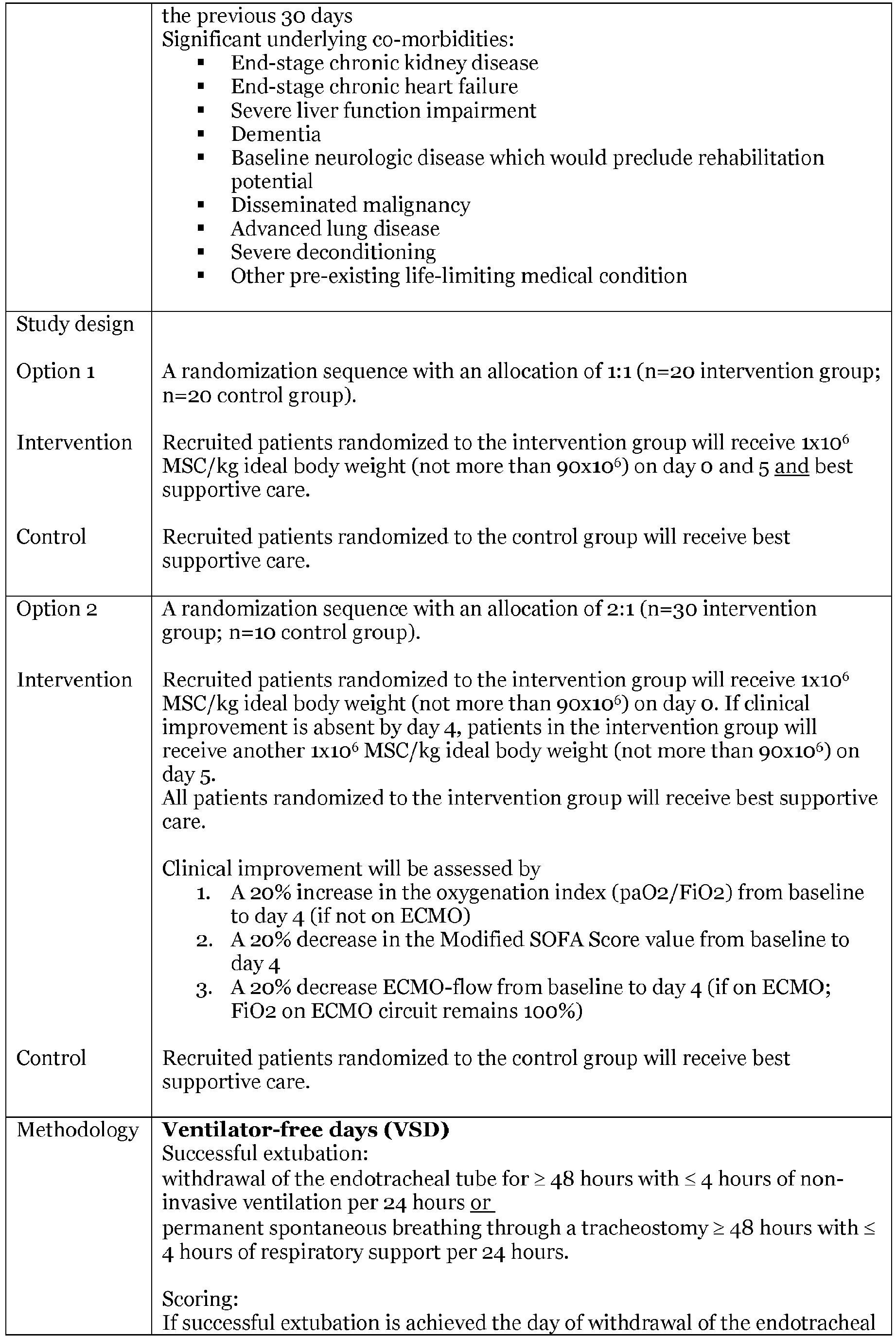

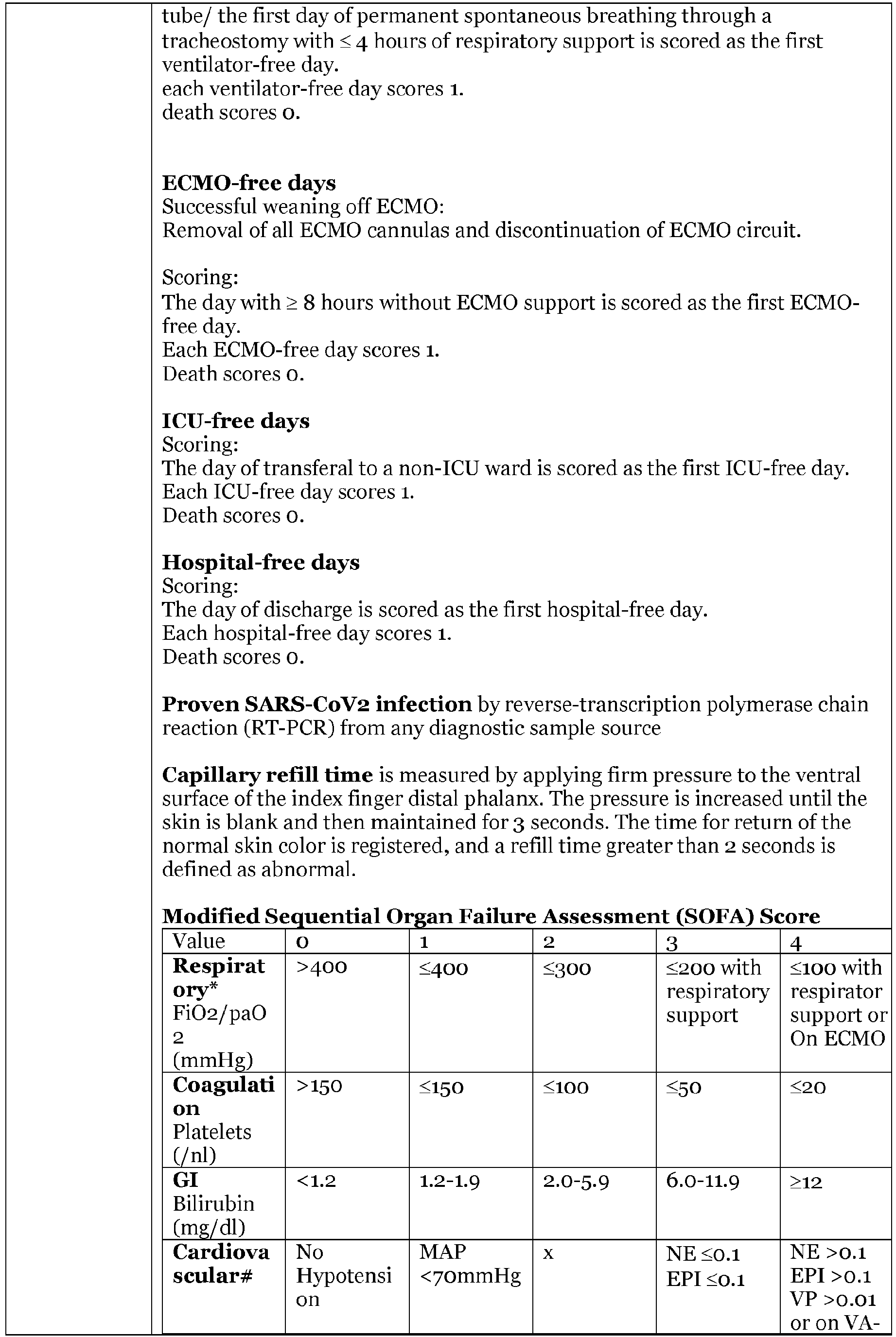

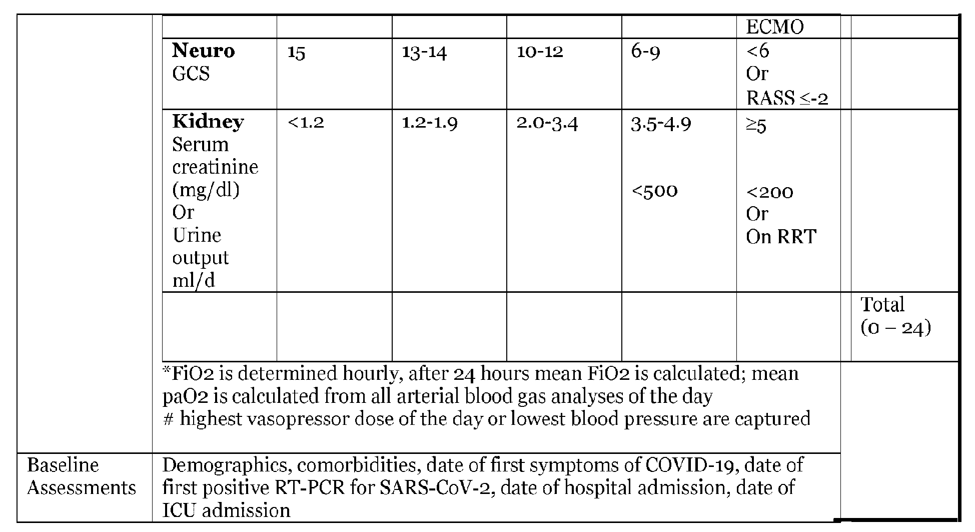

## Claims

1. **A cellular composition for use in the treatment of a disorder associated with a viral infection** in a subject, the cellular composition comprising mesenchymal stromal cells (MSC), and wherein the treatment comprises the administration of a therapeutically effective amount of the cellular composition to the subject.

2. The cellular composition for use of claim 1, wherein the viral infection in the subject is caused by a virus of the family of *Coronaviridae.*

3. The cellular composition for use of claim 1 or 2, wherein the virus infection is caused by HCoV-229E, HcoV-OC43 (HCoV-OC43), or severe acute respiratory syndrome coronavirus (SARS-CoV), and preferably is caused by SARS Cov-2.

4. The cellular composition for use of any one of claims 1 to 3, wherein the disorder is an inflammatory complication associated with a respiratory disorder caused by the viral infection in the subject.

5. The cellular composition for use of claim 4, wherein the inflammatory complication is a systemic inflammation and preferably is systemic endotheliitis.

6. The cellular composition for use of claim 5, wherein the presence of the endotheliitis in the subject is **characterized by** one or more of the following serum markers before administration of the MSC:
**(i)** Leucocytosis; preferably of 3 - 15, more preferably a leucocyte [plasma] count of 4 - 12, and most preferably 4.2 - 10.8 cells per nl;
**(ii)** Elevated c reactive protein levels, preferably of > about 5 mg/dl preferably of > about 10,5 mg/dl;
**(iii)** Normal or Elevated Interleukin (IL) - 6 levels;
**(iv)** Elevated HLA-DR activated cytotoxic CD8 positive T-cells;
**(v)** Optionally, normal or elevated procalcitonin (PCT) levels;
**(vi)** Normal or elevated lactat dehydrogenase (LDH) levels;
**(vii)** An increased capillary refill time, such as equal to, or more than, about 2 seconds, equal to, or more than, about 2.5 seconds, and preferably equal to, or more than, about than about 3 seconds.

7. The cellular composition for use of any one of claims 1 to 6, wherein the treatment comprises a reduction of a cell mediated immune response in the subject, preferably comprises a reduction of leucocytosis in the subject and preferably involves a reduction of activated CD3+/CD8+ T cells in the subject.

8. The cellular composition for use of any one of claims 1 to 7, wherein the subject is distinguished by showing signs of micro capillary failure, preferably defined as an increased capillary refill time, such as equal to, or more than, about 2 seconds, equal to, or more than, about 2.5 seconds, and preferably equal to, or more than, about 3 seconds.

9. The cellular composition for use of any one of claims 1 to 8, wherein the subject is distinguished by a pₐO₂/FᵢO₂ of less than about 250, 220, 200, 180 and preferably 150 mmHg while showing a positive end-expiratory pressure (PEEP) of equal to, or more than, 4, 5, 6, 7 and preferably 8 cmH₂O.

10. The cellular composition for use of any one of claims 1 to 9, wherein the subject is distinguished by an ongoing veno-venous / veno-arterial extra-corporal life support, preferably the subject is distinguished in that it receives extracorporeal membrane oxygenation (ECMO).

11. The cellular composition for use of any one of claims 1 to 10, wherein the MSC preparation is an allogenic and polygenic MSC preparation.

12. The cellular composition for use of any one of claims 1 to 11, wherein the therapeutically effective amount of MSC comprises 10⁵ to 10⁷, preferably about (+/- 20%) 1x10⁶ MSC/kg ideal body weight, and preferably not more than about 90x10⁶ MSC/kg body weight.

13. **A method for stratifying a subject suffering from a disorder associated with a viral infection for a treatment with mesenchymal stromal cells (MSC),** the method comprising
**(i)** Providing a biological sample of the subject;
**(ii)** determining in the biological sample one or more marker(s) of systemic inflammation,
wherein the subject is stratified for a treatment with MSC if the determined one ore more marker(s) of system inflammation indicates a systemic inflammation in the subject.

14. The method of claim 13, wherein the systemic inflammation is a systemic endotheliitis.

15. **A kit for stratifying a subject suffering from a disorder associated with a viral infection for a treatment with mesenchymal stromal cells (MSC),** comprising means for determining one or more marker(s) of systemic inflammation.
